# EUROPÄISCHE PATENTSCHRIFT

(11) **EP 3 930 787 B1**
(45) Veröffentlichungstag und Bekanntmachung des Hinweises auf die Patenterteilung: **04.12.2024**
(21) Anmeldenummer: 20707079.8
(22) Anmeldetag: 25.02.2020
(51) Int. Cl.: A61M 1/16, A61M 1/36

(54) **ÜBERWACHUNGSEINRICHTUNG FÜR REZIRKULATIONSBESTIMMUNG**
MONITORING SYSTEM FOR THE DETERMINATION OF RECIRCULATION
SYSTÈME DE SURVEILLANCE POUR LA DÉTERMINATION DE LA RECIRCULATION

(30) Priorität: 25.02.2019 DE 102019104738
(43) Veröffentlichungstag der Anmeldung: 05.01.2022
(73) Patentinhaber: B. Braun Avitum AG, 34212 Melsungen (DE)
(72) Erfinder: KRAUSE, Silvie, 34212 Melsungen (DE); WOLFF, Henrik, 34212 Melsungen-Adelshausen (DE); JAKOB, Marten, 37130 Klein Lengden (DE)
(74) Vertreter: Winter, Brandl - Partnerschaft mbB
(86) Internationale Anmeldenummer: PCT/EP2020/054833
(87) Internationale Veröffentlichungsnummer: WO 2020/173900

(56) Entgegenhaltungen:
- EP-A1- 2 783 716
- EP-B1- 2 783 715

## Beschreibung

Die vorliegende Offenbarung betrifft eine Überwachungseinrichtung einer extrakorporalen Blutbehandlungsmaschine, welche dazu ausgelegt ist, im Rahmen einer Dialysebehandlung ein Signal zu erfassen, das eine Schadstoffkonzentration in verbrauchter Dialyseflüssigkeit abbildet, sowie ein zugehöriges Überwachungsverfahren und eine extrakorporale Blutbehandlungsmaschine mit einer solchen Überwachungseinrichtung.

### Technischer Hintergrund

Bei extrakorporalen Blutbehandlungen, insbesondere Dialysebehandlungen, wird aus einem Blutgefäß eines Patienten an einer Blutentnahmestelle über einen Shunt Blut entnommen, in einer extrakorporalen Blutbehandlungsmaschine, insbesondere Dialysemaschine, gereinigt und anschließend stromabwärts der Blutentnahmestelle wieder über den Shunt in den Patienten eingeleitet. Es kommt vor, dass dabei bereits gereinigtes Blut in dem Shunt zu der Entnahmestelle zurückströmt und erneut in die extrakorporale Blutbehandlungsmaschine eingeleitet wird. D.h., es tritt eine Rezirkulation auf, durch welche das in die extrakorporale Blutbehandlungsmaschine eingeleitete, zu reinigende Blut durch rezirkuliertes, bereits gereinigtes Blut "verdünnt" wird und entsprechend das Ergebnis der Blutbehandlung sich verschlechtert. Demgemäß ist Rezirkulation ein wichtiges Thema bei extrakorporalen Blutbehandlungen. Ein Auftreten einer Rezirkulation sollte möglichst schnell und zuverlässig erkannt und Gegenmaßnahmen unmittelbar eingeleitet werden.

### Stand der Technik

Um eine Rezirkulation zweifelsfrei festzustellen, gibt es verschiedene punktuell durchführbare Messmethoden für Einzelmessungen, zum Beispiel ein auf dem Markt verfügbares System namens Transonic, welche jedoch nicht erlauben, eine Rezirkulation zuverlässig unmittelbar zu erkennen, sobald diese auftritt. Ferner gibt es Online-Messmethoden, welche jedoch einen hohen Kosten- und Geräteaufwand mit sich bringen.

Bei einer extrakorporalen Blutbehandlung wird neben einer Rezirkulation eine Vielzahl weiterer Parameter überwacht und eingestellt. Ein Beispiel hierfür ist die Überwachung der Behandlungseffektivität/des Behandlungsverlaufs bei einer extrakorporalen Blutbehandlungsmaschine, insbesondere Dialysemaschine, anhand eines Kt/V-Werts (mit K=Clearance, t=Dialysezeit, und V=Harnstoffverteilungsvolumen), welcher aus spektroskopischen Messungen berechnet wird und welcher die Reduktion der Stoffkonzentration harnpflichtiger Substanzen in der verbrauchten Dialyseflüssigkeit abbildet. Ein Beispiel hierfür ist das System Adimea von BBraun, welches Online-Informationen zu einem Behandlungsverlauf bereitstellt und eine hochgenaue Echtzeitmessung des Kt/V-Werts, insbesondere eines Kt/V-Adimea-Werts oder eines spKt/V-Werts (single spool Kt/V-Werts), während der Behandlung ermöglicht. Dazu ermittelt Adimea im Dialysatabfluss (also in der verbrauchten Dialyseflüssigkeit) eine UV-Absorption als Maß für die Harnstoffkonzentration im Plasma des Patienten, welche proportional zu der Konzentration von UV-absorptiven (harnstoffpflichtigen) Substanzen im Blut des Patienten ist. Diese Proportionalität tritt auch bei einem konstanten Grad von Rezirkulation auf, wobei sich jedoch ein entsprechender Proportionalitätsfaktor ändern kann.

Dementsprechend ist es bekannt, dass bestimmte Verhaltensmuster des Kt/V-Werts, insbesondere eines Kt/V-Adimea-Werts (beispielsweise ein plötzlicher Anstieg einer zugehörigen Kurve nach einer Reduzierung der Blutflussrate), auf eine vorliegende Rezirkulation hinweisen können. Allerdings ist der Kt/V-Wert von einer Vielzahl weiterer Faktoren abhängig, wie z. B. patientenspezifischen Parametern, diversen Problemen im Leitungssystem der Maschine, diversen Betriebsparametern der extrakorporalen Blutbehandlungsmaschine sowie der eingestellten und während einer Behandlung umstellbaren Dialysedosis. Ferner treten die auf eine Rezirkulation hinweisenden Verhaltensmuster entweder nur bei einer spezifischen Parameterveränderung auf, wie z.B. der vorstehend genannten Reduzierung der Blutflussrate, und erlauben somit keine Online-Messung, oder sind aufgrund der Vielzahl von darin einfließenden Parametern nicht oder nur schwer erkennbar. Der Stand der Technik sieht vor, wie vorstehend beschrieben entweder zusätzliche aufwändige Messgeräte für die Online-Messung einzusetzen oder beispielsweise in regelmäßigen Zeitabständen Einzelmessungen durchzuführen, welche zwar zuverlässig und genau sind, was jedoch zur Folge hat, dass eine Rezirkulation ggf. nicht unmittelbar erkannt werden kann. Aufgrund dessen hat sich die Entwicklung für Rezirkulation-Überwachungseinrichtungen bislang stark auf eine Verbesserung von Messgeräten für die Online-Messung konzentriert.

Beispielsweise ist aus EP 2 783 716 A1 ein Verfahren bekannt, welches die Erfassung einer Rezirkulation im Zugang eines Patienten oder einer Änderung einer Rezirkulation während einer laufenden Blutbehandlung mittels eines Dialysators ermöglicht, wobei während der Blutbehandlung aufeinanderfolgende Messwerte mittels eines Sensors am Abfluss des Dialysators ermittelt werden, und für einen jeweiligen, vom Sensor erzeugten Messwert oder eine hieraus gewonnene Größe ein Grenzwert vorgegeben wird. Wenn der Messwert oder die hieraus gewonnene Größe den Grenzwert erreicht oder überschreitet, wird eine Aktion wie etwa die Erzeugung eines optischen und/oder akustischen Warnhinweises eingeleitet. Weiterhin sind eine entsprechende Vorrichtung und ein entsprechendes Computerprogrammprodukt beschrieben.

EP 2 783 715 B1 offenbart ein Steuerungsverfahren einer Dialysemaschine zur Ermöglichung der dialysierflüssigkeitsseitigen Erfassung einer Rezirkulation in einem arteriovenösen Ein-/Auslass-Element, vorzugsweise Shunt, wobei die Dialysemaschine wenigstens einen Dialysierflüssigkeitseinlass sowie wenigstens einen Dialysierflüssigkeitsabfluss für eine ausgewählte Dialysierflüssigkeit und wenigstens eine Dialysierflüssigkeitspumpe hat, welche in fluidischer Verbindung mit wenigstens einem Dialysator stehen, wobei ein Sensor in Dialysierflüssigkeits-Strömungsrichtung gesehen dem Dialysator nachgelagert ist, der eine Änderung eines physikalischchemischen Parameters der abfließenden Dialysierflüssigkeit erfasst.

### Zusammenfassung der Erfindung

Die der Erfindung zugrunde liegende Aufgabe besteht darin, die Nachteile des Stands der Technik zu verringern. Insbesondere soll eine einfache, kostengünstige Überwachungseinrichtung für eine extrakorporale Blutbehandlungsmaschine bereitgestellt werden, die es ermöglicht, Verdachtsmomente hinsichtlich einer ggf. vorliegenden Rezirkulation automatisch, schnell und zuverlässig zu erkennen, um z.B. zu vermeiden, dass ein Patient langfristig eine zu geringe Dialysedosis erhält. Anschließend kann durch eine Einzelmessung verifiziert oder falsifiziert werden, ob tatsächlich eine Rezirkulation vorliegt.

Diese der Erfindung zugrunde liegende Aufgabe wird durch eine Überwachungseinrichtung mit den Merkmalen des Anspruchs 1 gelöst. Vorteilhafte Ausgestaltungen sind Gegenstand der Unteransprüche und werden später erläutert.

Bei einer Analyse der vorstehend beschriebenen Probleme der Rezirkulationsmessungen des Stands der Technik, wurde erkannt, dass diese auch behoben werden können, ohne dass ein Ermitteln einer Rezirkulation unmittelbar nach deren Auftreten (was durch Einzelmessungen nicht erzielbar ist) noch eine zuverlässige Bestimmung und Messung einer vorliegenden Rezirkulation (was anhand eines Kt/V-Signals nicht möglich ist) notwendig sind, um dennoch eine sowohl schnelle als auch zuverlässige Bestimmung einer Rezirkulation zu ermöglichen.

Die der Erfindung zugrunde liegende Aufgabe wird durch eine Überwachungseinrichtung einer extrakorporalen Blutbehandlungsmaschine gelöst, welche dazu ausgelegt ist, ein Signal zu erfassen, das (im Wesentlichen kontinuierlich) eine Schadstoffkonzentration in verbrauchter Dialyseflüssigkeit abbildet. Diese Überwachungseinrichtung ist ferner dazu ausgelegt, einen Verlauf des Signals oder Signalverlauf automatisch bezüglich zumindest eines vorbestimmten Indikators / Signalverlauf-Indikators / Signalverlauf-Musters für eine vorliegende Rezirkulation auszuwerten und bei Ermitteln des zumindest einen Indikators ein Auslösesignal auszugeben, durch welches automatisch eine Rezirkulationsmessung eingeleitet oder automatisch eine Aufforderung zum Einleiten einer Rezirkulationsmessung an einen Anwender ausgegeben wird. Eine Voraussetzung für die Auswahl des Signals sowie der das Signal erfassenden Messzelle (z.B. die Kt/V-Messzelle des Adimea-Systems) ist, dass das Signal sehr stabil und zuverlässig messbar sein muss. Eine Rezirkulationsmesszelle, welche dazu vorgesehen ist, die Rezirkulationsmessung vorzugsweise als eine Einzelmessungen durchzuführen, kann eine vorliegende Rezirkulation genau und zuverlässig bestimmen oder verifizieren. Somit kann vorteilhafterweise eine auftretende Rezirkulation unmittelbar erkannt werden und kann vermieden werden, dass ein Patient langfristig eine zu geringe Dialysedosis erhält.

Die Überwachungseinrichtung ist ferner dazu ausgelegt, das Signal zu differenzieren, um ein Differenzialsignal zu berechnen. Dieses Differenzialsignal kann anschließend mit zumindest einem Schwellwert verglichen werden, wobei als einer der Indikatoren für eine vorliegende Rezirkulation eine bestimmte Häufigkeit (z.B. häufiger als drei- oder viermal) oder Dauer von Schwellwertüberschreitungen vorzugsweise innerhalb eines bestimmten Zeitraums nutzbar ist. Auf diese Weise kann insbesondere ein springender Signalverlauf und ggf. eine deutlich stärkere Abnahme der Schadstoffkonzentration als erwartet erkannt werden. Alternativ oder zusätzlich kann eine einmalige Schwellwertüberschreitung des gleichen oder eines anderen, höheren Schwellwerts als einer der Indikatoren für eine vorliegende Rezirkulation erkannt werden. Dadurch lässt sich insbesondere ein plötzlich auftretender, hoher Anstieg des Signalverlaufs erkennen. Alternativ oder zusätzlich kann auch eine bestimmte Häufigkeit oder Dauer von Unterschreitungen eines weiteren, unteren Schwellwerts vorzugsweise innerhalb eines bestimmten Zeitraums als Indikator verwendet werden, was entsprechend auf einen springenden Signalverlauf sowie ggf. auf eine deutlich schwächere Abnahme der Schadstoffkonzentration als erwartet zurückgeführt werden kann. Demgemäß können aus einer einfachen Differenzierung des Signals (aus dem Differenzialsignal) verschiedene Indikatoren für eine auftretende Rezirkulation abgeleitet werden, was eine einfache, wenig Rechenleistung benötigende Operation ist.

Der/die Schwellwert/e sind von der Soll-Behandlungszeit und einem Signalwert einer oder mehrerer früherer extrakorporaler Blutbehandlung(en) des spezifischen Patienten, beispielsweise von einem Durchschnitt solcher Signalwerte, abhängig. Auf diese Weise können auch patientenspezifische Parameter berücksichtigt werden. Als Referenz zur Berechnung des Schnellwerts/der Schwellwerte können beispielsweise Kt/V-Werte zu bestimmten Zeitpunkten, ein Kt/V-Signalverlauf bzw. dessen Form, eine Anzahl von Steigungsänderungen des Signalverlaufs, eine Höhe von Abweichungen zwischen verschiedenen Signalverläufen, eine typische Variabilität für den spezifischen Patienten, Durchschnitte o.Ä. berücksichtigt werden. Beispielsweise kann eine Anzahl an akzeptablen Signalsprüngen eines Kt/V-Signals des spezifischen Patienten angepasst werden, beispielsweise wenn er bekanntermaßen häufig unruhig ist, ohne dass bislang eine Rezirkulation ermittelt wurde.

In anderen Worten wurden Signalverläufe, welche die Schadstoffkonzentration in verbrauchter Dialyseflüssigkeit bei einer extrakorporalen Blutbehandlung abbilden, insbesondere hinsichtlich auftretenden Rezirkulationen analysiert und daraus verschiedene Indikatoren aus dem Signalverlauf abgeleitet, welche ein Indiz dafür sein können, dass bei dem aktuell behandelten Patienten eine Rezirkulation vorliegt. Beispielsweise kann der Signalverlauf eine deutlich geminderte Dialyseeffizienz anzeigen, d.h., eine deutlich langsamer als erwartet sinkende Schadstoffkonzentration, wenn eine konstante Rezirkulation vorliegt. Dies ist dadurch begründbar, dass das die extrakorporale Blutbehandlungsmaschine durchlaufende, zu reinigende Blut durch das bereits gereinigte, rezirkulierte Blut verdünnt wird und somit verhältnismäßig wenig Schadstoffe entfernt werden können. Andererseits kann der Signalverlauf den Anschein erwecken, die Schadstoffkonzentration sinke deutlich schneller als erwartet bzw. die Dialyseeffizienz sei deutlich höher als erwartet, was auf eine zunehmende Rezirkulation von bereits gereinigtem Blut hinweisen kann. Des Weiteren kann ein unsteter, sprung behafteter Signalverlauf auftreten, was beispielsweise auf Turbulenzen im Shunt, durch welchen die extrakorporale Behandlungsmaschine an den Patienten angeschlossen ist, oder auf patientenbedingte Parameter wie ein häufiges Bewegen des Patienten zurückführbar sein kann. Derartige Turbulenzen oder häufige Patientenbewegungen können ebenfalls eine Rezirkulation begünstigen. Demgemäß kann auch ein "springender", unsteter Signalverlauf auf eine Rezirkulation hinweisen. Ferner kann bei einer einsetzenden Rezirkulation ein einzelner, hoher Anstieg des Signalverlaufs auftreten. Diese auf eine Rezirkulation hinweisenden Signalverläufe können einander selbstverständlich auch überlagern.

Die somit ermittelten Indikatoren wurden in der erfindungsgemäßen Überwachungseinrichtung, insbesondere einer Speichereinheit derselben, hinterlegt bzw. gespeichert, sodass diese automatisch die Indikatoren während der Signal-Messung isolieren und erkennen kann. Da die Indikatoren jedoch auch eine Vielzahl anderer Ursachen haben können, wird anschließend, nach Ermitteln des vorliegenden Indikators, ein Trigger oder ein Auslösesignal ausgegeben, der bzw. das z.B. eine genaue Einzelmessung automatisch einleitet und vorzugsweise ein Warnsignal ausgibt, wenn ein Vorliegen einer Rezirkulation bestätigt ist. Alternativ wird durch den Trigger oder das Auslösesignal automatisch ein Hinweis für einen Anwender ausgegeben, dass eine derartige Einzelmessung manuell eingeleitet werden soll. Auf diese Weise ist es möglich, die Unmittelbarkeit der Messung des Signals zu nutzen, ohne auf eine zuverlässige Bestimmung der Rezirkulation verzichten zu müssen. Anschließend werden vorzugsweise Ergebnisse der somit eingeleiteten oder ausgelösten Rezirkulations- oder Einzelmessungen in einer Historie für eine automatische oder manuelle Auswertung oder Kontrolle gespeichert werden. Dabei werden weiter vorzugsweise automatisch getriggerte/eingeleitete Messungen von manuell getriggerten/eingeleiteten Messungen z.B. durch unterschiedliche Farbe oder Marker dargestellt.

Vorzugsweise bildet das Signal einen Kt/V-Wert, insbesondere einen mit Adimea ermittelten Kt/V-Adimea-Wert, ab oder ist ein Rohsignal, vorzugsweise Intensitäts- und/oder Absorptionswerte einer spektroskopischen Messung, auf dessen Basis der Kt/V-Wert berechenbar ist. Der Kt/V-Wert wird bei extrakorporalen Blutbehandlungen standardmäßig auf verschiedene Weise ermittelt, beispielsweise über das Adimea-System von BBraun, welches eine sehr stabile, zuverlässige Online-Messung des Kt/V-Signals ermöglicht.

Vorzugsweise ist der Schwellwert oder sind die Schwellwerte abhängig von einer Soll-Behandlungszeit sowie einem Soll-Signalwert und/oder einem theoretisch errechneten Signalwert. Beispielsweise kann hierfür ein Kt/V-Watson-Wert für die komplette Behandlung oder Teile der Behandlung herangezogen werden. Bei dem Kt/V-Watson-Wert wird das Harnstoffverteilungsvolumen (V) nach einer sogenannten Watson-Formel berechnet, wobei die Clearance (K) entweder basierend auf Eigenschaften des Dialysators und/oder des Bluts und ggf. des Dialyseflüssigkeitsstroms oder anhand einer Tabelle o.Ä. bestimmt wird. D.h., der Signalverlauf wird mit einem idealen oder theoretischen Signalverlauf verglichen und ein bestimmter Grad an Abweichung von diesem idealen oder theoretischen Signalverlauf wird als Indikator für eine vorliegende Rezirkulation genutzt.

Weiter bevorzugt kann die Überwachungseinrichtung dazu eingerichtet sein, aus dem Signal eine erwartete Behandlungszeit zu errechnen, zu welcher ein Soll-Signalwert voraussichtlich erreicht wird, und mit einer Soll-Behandlungszeit zu vergleichen, wobei ein deutliches Unterschreiten und/oder ein deutliches Überschreiten der Soll-Behandlungszeit als ein Indikator für eine vorliegende Rezirkulation dient. In anderen Worten wird ein Schnittpunkt des Signals basierend auf dessen mittlerer Steigung mit einem Soll-Signalwert oder einer Soll-Schadstoffkonzentration berechnet. Ein deutliches Unterschreiten (beispielsweise eine erwartete Behandlungszeit von 60% der Soll-Behandlungszeit) zeigt, dass die Schadstoffkonzentration deutlich schneller sinkt als erwartet, und ein deutliches Überschreiten zeigt, dass die Schadstoffkonzentration deutlich langsamer sinkt als erwartet. Dies ist insbesondere Vorteilhaft, da über diese Überwachung der erwarteten Behandlungszeit auch deutlich sprungbehaftete/springende Signalverläufe einfach hinsichtlich ihrer mittleren Steigung bzw. hinsichtlich der erwarteten Behandlungsdauer bewertbar sind.

Alternativ oder zusätzlich kann es von Vorteil sein, den Signalverlauf mit bekannten, vorab abgespeicherten Mustern oder Pattern eines Signalverlaufs zu vergleichen, welche bei einer vorliegenden Rezirkulation auftreten, und als Indikator für eine vorliegende Rezirkulation eine ausreichender Übereinstimmung des Signals mit den bekannten Mustern oder Pattern zu erkennen. Derartige vorab gespeicherte Muster oder Pattern können Signalverläufe oder Templates sein, welche für eine Rezirkulation typisch sind, z.B. mit einem plötzlichen, hohen Anstieg oder häufigen Sprüngen. Die Überwachungseinrichtung ist in diesem Fall dazu eingerichtet, den Signalverlauf häufig oder kontinuierlich mit diesen Mustern oder Pattern zu vergleichen, beispielsweise mittels einer Kreuzkorrelationsberechnung, und eine ausreichende Übereinstimmung als einen der Indikatoren für eine vorliegende Rezirkulation zu erkennen. Diese Vorgehensweise ist insbesondere Vorteilhaft, wenn das Rohsignal, insbesondere ein Intensitäts- und/oder Absorptionssignal, zur Ermittlung des Indikators verwendet wird, da auf diese Weise auf einige ggf. rechenaufwändige Signalverarbeitungsschritte verzichtet werden kann.

Nach einem Aspekt der vorliegenden Erfindung kann die Überwachungseinrichtung dazu ausgelegt sein, nach dem Ermitteln des zumindest einen Indikators, vorzugsweise unmittelbar danach, eine Plausibilitätsprüfung einzuleiten. D.h., eine zusätzliche Prüfung wird eingeleitet, sobald ein vorliegender Indikator ermittelt wurde, bei welcher geprüft wird, ob der Indikator tatsächlich auf eine mögliche Rezirkulation hinweisen kann bzw. ob deren Vorliegen wahrscheinlich ist, oder ob es ggf. andere Parameter gibt, die das Auftreten des Indikators ausgelöst haben könnten. Dies ist vorteilhaft, da auf dieses Weise unnötige Rezirkulationsmessungen vermieden werden können, wodurch der Verlauf der extrakorporalen Blutbehandlung und/oder dessen Überwachung weniger gestört wird.

Vorzugsweise kann im Rahmen der Plausibilitätsprüfung das Auftreten des Indikators mit weiteren Sensordaten, vorzugsweise einem venösen Druck, verglichen werden. Tritt beispielsweise ein häufig springender Signalverlauf (insbesondere einer Kt/V-Kurve) auf, ggf. gleichzeitig mit starken, durch entsprechende Drucksensoren erfassten Schwankungen des arteriellen und venösen Drucks, werden Druckmesswerte eines venösen Drucksensors analysiert. Überschreiten diese Druckmesswerte einen definierten, ggf. patientenindividuellen Grenzwert, wie z.B. 150mmHg, ggf. unmittelbar nachdem solche Signalverläufe beobachtet wurden oder gleichzeitig dazu, ist dies ein zusätzlicher Hinweis auf eine Veränderung am Gefäßzugang oder auf eine suboptimale Nadellage. Demgemäß ist in diesem Fall ein Plausibilitätsprüfungsergebnis positiv und wird eine Rezirkulationsmessung automatisch eingeleitet oder wird eine entsprechende Aufforderung an den Anwender automatisch ausgegeben.

Alternativ oder zusätzlich kann das Auftreten des Indikators mit einer Änderung eines Betriebszustands oder Parameters der extrakorporalen Blutbehandlungsmaschine verglichen werden. Beispielsweise wird geprüft, ob (insbesondere unmittelbar) vor dem Auftreten des Indikators ein Betriebsparameter geändert wurde und ob die dem Indikator zugehörige Änderung des Signalverlaufs auf diesen geänderten Betriebsparameter zurückführbar ist, was zu einem negativen Plausibilitätsprüfungsergebnis führen würde. Weiter beispielsweise kann es den Verdacht auf eine Rezirkulation verstärken und somit ein positives Plausibilitätsprüfungsergebnis bewirken, wenn der Indikator eines deutlich zu großen Schadstoffkonzentrationsabfall unmittelbar auf eine vorhergehende Reduzierung der Blutflussrate folgt.

Ferner ist es möglich, die Überwachungseinrichtung derart zu konfigurieren, dass sie mehrere der Indikatoren ermittelt und diese im Rahmen der Plausibilitätsprüfung vergleicht. Also prüft in diesem Fall die Überwachungseinrichtung, ob mehrere der vorstehend beschriebenen Indikatoren gleichzeitig auftreten, wie beispielsweise ein häufig springender Signalverlauf in Kombination mit einem deutlich zu schnellen oder zu langsamen Sinken der Schadstoffkonzentration, und gibt ein positives Plausibilitätsprüfungsergebnis aus, wenn dies der Fall ist, oder gibt alternativ ein negatives Plausibilitätsprüfungsergebnis aus, wenn kein weiterer Indikator ermittelt wurde. Diese Art der Plausibilitätsprüfung kann basierend auf ausschließlich dem Signalverlauf durchgeführt werden. D.h., es ist kein Abgleich mit weiteren Sensoren oder der Steuerung der extrakorporalen Blutbehandlungsmaschine notwendig.

Vorzugsweise ist die Überwachungseinrichtung ferner dazu angepasst, in einem Fall, in welchem die Rezirkulationsmessung ergibt, dass keine Rezirkulation vorliegt, weitere Messungen einzuleiten, um ggf. andere Ursachen für das Vorliegen des zumindest einen Indikators zu ermitteln.

Ferner wird die der Erfindung zugrunde liegende Aufgabe durch ein Überwachungsverfahren einer Überwachungseinrichtung gelöst, welche der vorstehend beschriebenen erfindungsgemäßen Überwachungseinrichtung entspricht. Das Überwachungsverfahren weist folgende Schritte auf: Erfassen eines Signals, das eine Schadstoffkonzentration in verbrauchter Dialyseflüssigkeit abbildet, automatisches Auswerten eines Verlaufs des Signals bezüglich zumindest eines vorbestimmten Indikators für eine vorliegende Rezirkulation und automatisches Einleiten einer Rezirkulationsmessung oder Ausgeben einer automatischen Aufforderung zum Einleiten einer Rezirkulationsmessung an einen Anwender, wenn der zumindest eine Indikator ermittelt wurde, insbesondere über einen Trigger oder ein Auslösesignal. Vorzugsweise kann ferner ein Schritt vorgesehen sein, gemäß welchem eine Plausibilitätsprüfung eingeleitet wird, nachdem, vorzugsweise unmittelbar nachdem, der zumindest eine Indikator ermittelt wurde.

Darüber hinaus wird die der Erfindung zugrunde liegende Aufgabe gelöst durch eine extrakorporale Blutbehandlungsmaschine, vorzugsweise eine Dialysemaschine, mit einer vorstehend beschriebenen Überwachungseinrichtung.

Hinsichtlich der genaueren Ausgestaltung des vorstehend beschriebenen Überwachungsverfahrens sowie der extrakorporalen Blutbehandlungsmaschine wird auf die vorstehende Beschreibung der erfindungsgemäßen Überwachungseinrichtung verwiesen.

In anderen Worten ausgedrückt, wirkt sich eine auftretende Rezirkulation direkt auf den Verlauf einer Kt/V-Kurve (Kt/V als f(t)) aus. Da dieser Verlauf vorzugsweise durch das Adimea-System überwacht wird, kann eine Abweichung von dem erwarteten Verlauf der Kurve durch Rezirkulation bedingt sein. Hinweise für eine auftretende Rezirkulation können z.B. Sprünge im Kurvenverlauf, ein sehr flacher, monotoner Verlauf, eine zu große Steigung, o.Ä. sein. Als ein Plausibilitätscheck können verschiedene Maßnahmen dienen, wie ein Vergleich mit gespeicherten Kurvenverläufen, ein Vergleich mit nach einem anderem Verfahren (z.B. Kt/V-Watson) bestimmten Werten, zusätzlich ein Abgleich mit dem aktuellen Maschinenzustand, o.Ä.. Die Ableitung der Kt/V-Kurve, insbesondere der spKt/V-Kurve, kann in Zusammenhang mit einem definierten Schwellwert genutzt werden, um einen 'unsteten' Anstieg zu ermitteln. Ein mehrmaliges Überschreiten des Schwellwertes dient dann als Trigger für eine Rezirkulationsmessung.

Oder nochmals anders ausgedrückt, kann man während der Behandlung laufend die Kt/V-Kurve, insbesondere spKt/V-Kurve, bzw. deren Ableitungen überwachen und kann man diese Ableitung nutzen, um anhand eines Schwellwertes einen unsteten Anstieg der Kurve zu testen. Peaks oder Spitzenwerte, die den Schwellwert überschreiten, können gezählt werden. Sollte der Schwellwert mehrfach, z.B. öfter als drei- oder viermal, überschritten werden, dann wird durch die Maschine eine Warnung ausgegeben, dass evtl. eine Rezirkulation vorliegt oder es kann automatisch eine Messung dieser Rezirkulation durch die Maschine eingeleitet werden. Eine sinnvolle Annahme für den Schwellwert kann aus einer gewünschten Behandlungszeit und einem Ziel-Kt/V-Wert bestimmt werden, oder aus einer (gewünschten) Behandlungszeit und einem Kt/V-Wert früherer Behandlungen, oder aus einer (gewünschten) Behandlungszeit und einen theoretisch über ein Watson-V errechneten Kt/V-Wert, wobei der der Dialysator bekannt sein muss.

Ein ähnliches Vorgehen ist mit den Rohsignalen, d.h. einer Intensität oder Absorbance (Absorptionswert), ebenfalls möglich. Ebenso könnte die Suche nach bestimmten Patterns (Mustern) unter Nutzung von bekannten Abstandmaßen verwendet werden, um bestimmte Features oder Indikatoren innerhalb der jeweiligen Kurven zu detektieren. Zusätzlich könnte die Verknüpfung von Features zu einer Erhöhung der Robustheit der Triggerung beitragen, sowie der Abgleich mit bestimmten anderen Zustandsgrößen der Maschine, da mitunter auch durch Parameteränderungen an der Maschine bestimmte Features erzeugt werden. Ein Abgleich des Maschinenzustandes für den Zeitpunkt, zu dem ein Feature detektiert wird, kann hier zusätzlich als eine Plausibilitätsprüfung dienen.

Beispiele für weitere Features sind nachfolgend beschrieben: Tritt eine sprungbehaftete/springende Kt/V-Verlaufskurve auf und beispielsweise ein venöser Druck, der ebenfalls einen definierten, ggf. patientenindividuellen Grenzwert überschreitet (z.B. 150 mmHg), dann wäre dies ein zusätzlicher Hinweis auf eine Veränderung am Gefäßzugang. Stark erhöhte venöse Drücke können ein Indikator für Abflussstenosen sein. Wenn keine starken Variationen der Kt/V-Kurve vorliegen, aber ihr Anstieg deutlich steiler verläuft als der zu erwartende Anstieg für den jeweiligen Patienten, kann auch dies als Triggerevent (also als Auslöser für das Ausgeben des Triggers oder Auslösesignals) dienen. D.h., die Kt/V Kurve kann deutlich oberhalb eines prognostizierten, linearen Behandlungsverlaufs (Kt/V-Signalverlaufs) liegen, sodass das Kt/V-Ziel von beispielsweise 1.2 scheinbar schon nach vermeintlich 60% der Behandlungszeit erreicht wird. Als Triggergröße (Indikator) könne in so einem Fall das Überschreiten eines absoluten Schwellwerts für den Anstieg der Kt/V-Kurve/des Kt/V-Signalverlaufs dienen. Alternativ könnte eine Bestimmung des Schnittpunktes der Soll-Kt/V-Kurve mit der Ist-Kt/V-Kurve oder der prädizierten Kt/V-Kurve (aus den aktuell vorhandenen Kt/V-Daten extrapoliert) als Kriterium dienen. Liegt dieser Schnittpunkt auf der Zeitachse deutlich vor der veranschlagten Zeit, so kann dies als Indiz dienen, dass eine signifikante Rezirkulation vorliegt. Da Patientenbewegungen zu Schwankungen in Drücken, aber auch zu Schwankungen in der Kt/V Kurve führen können, bietet eine zusätzliche Analyse von zeitgleich auftretenden, großen Schwankungen eines arteriellen und venösen Drucks ein Indiz für eine solche Bewegung. Wird anschließend ein hoher venöser Druck, ein Peak/eine Singalspitze in der Ableitung der Kt/V-Kurve/dem Differenzialsignal o.Ä. beobachtet, kann eine Rezirkulationsmessung eingeleitet werden.

### Figurenbeschreibung

Nachfolgend wird eine Ausführungsform der vorliegenden Erfindung anhand der beigefügten Figuren genauer beschrieben. Diese sind lediglich veranschaulichender Natur und sollen den Umfang der vorliegenden Erfindung nicht beschränken.
Fig. 1a bis Fig. 1f zeigen Kt/V-Signalverläufe nach einer Ausführungsform der Erfindung, welche auf eine vorliegende Rezirkulation hinweisen können.
Fig. 2a zeigt einen typischen, rezirkulationslosen spKt/V-Signalverlauf und dessen Ableitung und Fig. 2b zeigt einen springenden spKt/V-Signalverlauf und dessen Ableitung.
Fig. 3 zeigt schematisch eine extrakorporale Blutbehandlungsmaschine mit einer erfindungsgemäßen Überwachungseinrichtung.
Fig. 4 zeigt ein Flussdiagramm, welches einen Verfahrensablauf zur Ermittlung und Verarbeitung eines Rezirkulationsindikators veranschaulicht.

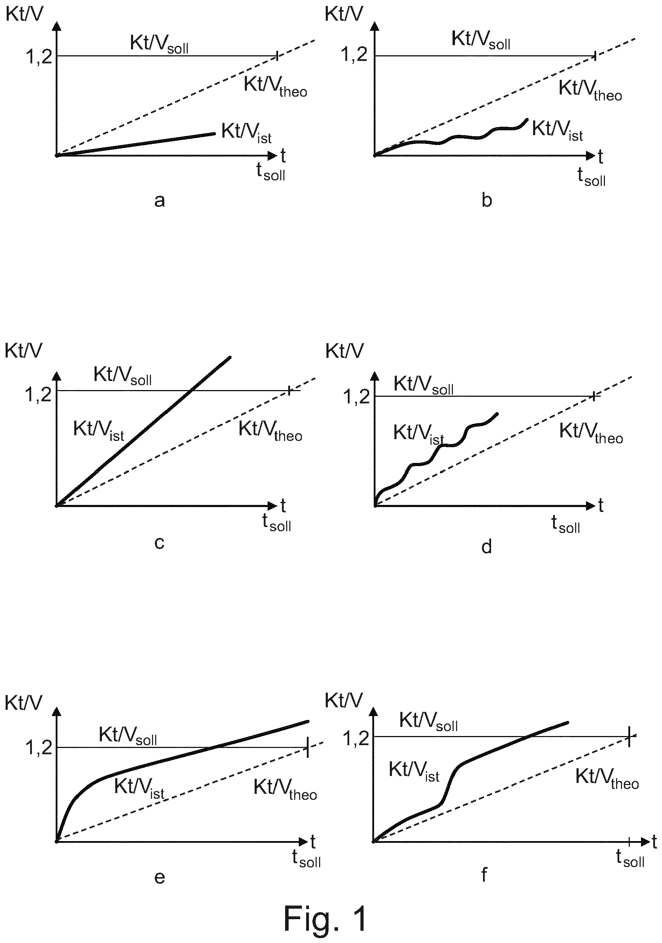

Fig. 1a bis Fig. 1f zeigen jeweils ein Diagramm, in welchem ein Verlauf eines Kt/V-Signals Kt/V_{Ist} über der Zeit t als eine durchgezogene Linie dargestellt ist. Ferner ist in dem Diagramm ein Soll-Kt/V-Wert Kt/V_{Soll} von 1,2 gekennzeichnet. Eine gestrichelte Linie zeigt einen theoretischen, linearen Kt/V-Verlauf Kt/Vtneo an, bei welchem der Soll-Kt/V-Wert Kt/V_{Soll} bei einer bestimmten Soll-Behandlungszeit t_{Soll} erreicht wird. Diese Figuren unterscheiden sich wie nachfolgend beschrieben hinsichtlich der dargestellten Signalverläufe.

Fig. 1a zeigt einen flachen Signalverlauf des Kt/V-Signals Kt/V_{Ist}. Das Kt/V-Signal Kt/Vist verläuft deutlich flacher als der theoretische Kt/V-Verlauf Kt/Vtneo und der Soll-Kt/V-Wert Kt/V_{Soll} wird voraussichtlichtlich nicht innerhalb der Soll-Behandlungszeit t_{Soll} erreicht. Dies kann ein Hinweis auf eine vorliegende Rezirkulation sein, da in diesem Fall das den Filter durchströmenden Blut, welches tatsächlich gereinigt werden muss, durch das bereits gereinigte, rezirkulierte Blut verdünnt wird. Auf diese Weise nimmt eine Konzentration von in dem Blut gelösten Schadstoffen deutlich langsamer ab als erwartet, was sich in der verbrauchten Dialyseflüssigkeit z.B. mittels des Adimea-Systems von BBraun messen lässt. Demgemäß dient das in Fig. 1a dargestellte Kt/V-Signal Kt/V_{Ist} als ein Indikator für eine vorliegende Rezirkulation. Fig. 1b zeigt einen flachen Signalverlauf, ähnlich wie in Fig. 1a, welcher zusätzlich sprungbehaftet oder unstet verläuft. Ein Signalverlauf ohne Sprünge wie in Fig. 1a tritt häufiger bei einer Behandlung mit einem an den Patienten angeschlossenen Katheter auf, als bei extrakorporalen Blutbehandlungen. Der in Fig. 1b dargestellte, unstete Verlauf kann auf Turbulenzen in einem Shunt hinweisen, über welchen ein Patient an der extrakorporalen Blutbehandlungsmaschine angeschlossen ist, oder kann auf häufige Bewegungen des Patienten hinweisen. Auch dies kann sowohl in Kombination mit dem flachen Signalverlauf als auch als ein alleinstehendes Merkmal auf eine bestehende Rezirkulation hinweisen und als Indikator für eine solche dienen. In anderen Worten ausgedrückt, wird eine konstante Rezirkulation durch eine springende Kurve und ein schlechte Behandlungsergebnis angezeigt werden. Dies kann durch eine Beobachtung von sich wiederholenden, kurzfristigen Änderungen der Steigung der Kurve und das schlechte Behandlungsergebnis (bei einem längerfristigen Trend bzw. einer Prognose des Signals oder Kt/V-Werts am Ende der Behandlung) identifiziert werden.

Fig. 1c zeigt einen stark ansteigenden Signalverlauf des Kt/V-Signals Kt/V_{Ist}. Das Kt/V-Signal Kt/V_{Ist} verläuft deutlich steiler als der theoretische Kt/V-Verlauf Kt/Vₜₕₑₒ und der Soll-Kt/V-Wert Kt/V_{Soll} wird voraussichtlichtlich schon deutlich vor Erreichen der Soll-Behandlungszeit t_{Soll} erreicht. Dies kann ein Hinweis auf eine vorliegende, zunehmende Rezirkulation sein, da in diesem Fall immer mehr bereits gereinigtes Blut durch den Shunt zurück in die extrakorporale Blutbehandlungsmaschine strömt, sodass das den Filter durchströmende Blut, welcher noch gereinigt werden muss, zunehmend durch bereits gereinigtes, rezirkuliertes Blut verdünnt wird. Auf diese Weise ermittelt eine Kt/V-Messzelle, welche das Kt/V-Signal Kt/V_{Ist} misst, eine stetige Abnahme der Schadstoffkonzentration, welche grundsätzlich auf eine erfolgreiche extrakorporale Blutbehandlung hindeutet, obwohl die Abnahme der Schadstoffkonzentration hauptsächlich auf die zunehmende Rezirkulation zurückzuführen ist und das tatsächliche Behandlungsergebnis sich zunehmend verschlechtert. Dies fällt meist dadurch auf, dass die extrakorporale Blutbehandlung deutlich effizienter erscheint als erwartet. Demgemäß kann auch ein deutlich zu stark ansteigender Kt/V-Signalverlauf ein Indikator für eine vorliegende Rezirkulation sein. Fig. 1d zeigt einen steilen Signalverlauf gemäß Fig. 1c, welcher zusätzlich, wie bereits im Rahmen von Fig. 1b genauer beschrieben, sprungbehaftet oder unstet verläuft, was sowohl alleinstehend als auch in Kombination mit einem steilen Signalverlauf als Indikator für eine vorliegende Rezirkulation dienen kann. In anderen Worten ausgedrückt, wird eine zunehmende Rezirkulation durch ein springendes Signal und ein gutes Behandlungsergebnis angezeigt, was den zunehmenden Grad der Rezirkulation abbildet. Indikatoren sind also kurzfristige Änderungen in der Steigung des Signalverlaufs und das zu gute Behandlungsergebnis (bei einem längerfristigen Trend bzw. einer Prognose des Signals oder Kt/V-Werts am Ende der Behandlung).

Fig. 1e und Fig. 1f zeigen jeweils ein Kt/V-Signal Kt/V_{Ist}, in dessen Verlauf ein plötzlicher, starker Anstieg auftritt. Bei Fig. 1e tritt dieser Anstieg zu Beginn der Messung auf, während er bei Fig. 1f erst in deren Verlauf auftritt. Ein solcher plötzlicher, starker Anstieg des Kt/V-Signals Kt/V_{Ist} kann darauf zurückgeführt werden, dass im Fall einer Rezirkulation das zu reinigende Blut plötzlich durch das rezirkulierte, bereits gereinigte Blut verdünnt wird. Demgemäß sinkt die Schadstoffkonzentration im Blut, was in einem plötzlichen Anstieg des Kt/V-Werts resultiert, der aus der Vermessung der verbrauchten Dialyseflüssigkeit ermittelt wird. Somit kann auch ein solcher plötzlicher, starker Antieg des Kt/V-Signals als Indikator für eine vorliegende Rezirkulation dienen.

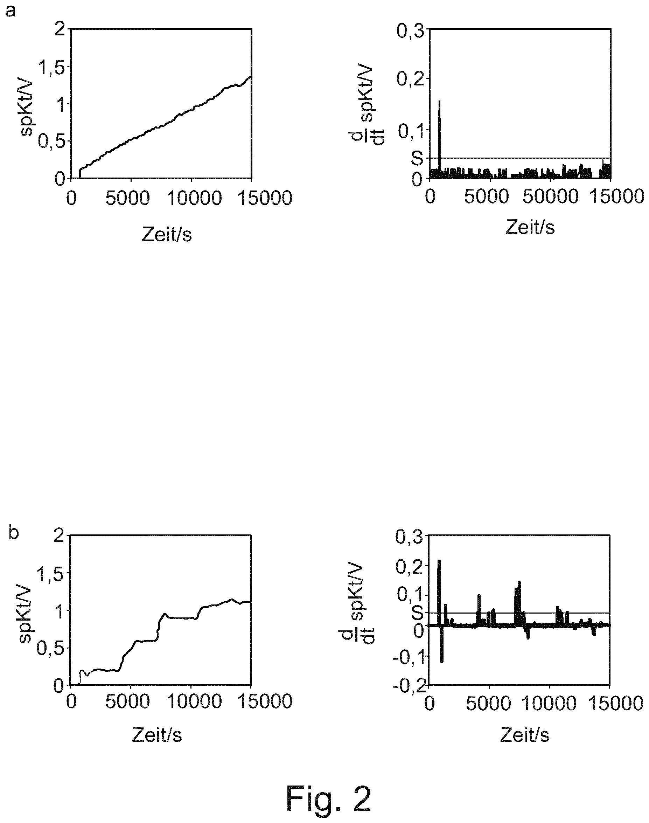

Fig. 2a zeigt ein typisches, rezirkulationsloses Kt/V-Signal (links), insbesondere ein spKt/V-Signal, wie es beispielsweise mittels Adimea von BBraun aufgenommen wird, und dessen Ableitung (rechts), wobei die Ableitung in regelmäßigen, verhältnismäßig kurzen Zeitintervallen berechnet wurde. Dabei ist zu erkennen, dass das rezirkulationsloses Kt/V-Signal im Wesentlichen linear ansteigt. Lediglich zu Beginn der Messung sowie gegen Ende des hier dargestellten spKt/V-Signalverlaufs treten Unregelmäßigkeiten auf, wobei erstere auf ein leicht verspätetes Einsetzen der Messung zurückführbar ist. Diese Unregelmäßigkeiten werden in der Ableitung des spKt/V-Signals, welche ansonsten im Wesentlichen gleichmäßig, mit einer mittleren Steigung von Null verläuft, als Signalspitzen oder Peaks abgebildet. Ein Schwellwert S ist derart festgelegt, dass die Ableitung bei einem theoretischen bzw. linearen spKt/V-Signalverlauf stets darunter liegt. Die beiden in Fig. 2a auftretenden Signalspitzen der Ableitung des spKt/V-Signals (des Differenzialsignals) erreichen oder überschreiten diesen Schwellwert S.

Fig. 2b zeigt das entsprechende Kt/V-Signal (links), insbesondere das spKt/V-Signal, in einem Fall, in welchem möglicherweise eine Rezirkulation vorliegt, sowie die Ableitung (rechts) des Signals, wobei die Ableitung in regelmäßigen, verhältnismäßig kurzen Zeitintervallen berechnet wurde. Dabei ist zu erkennen, dass das Kt/V-Signal in diesem Fall stark springt. Diese Sprünge oder Unregelmäßigkeiten resultieren in einer stark fluktuierenden Ableitung des spKt/V-Signals mit einer Vielzahl von sowohl positiven als auch negativen Signalspitzen oder Peaks. Der Schwellwert S, welcher derart festgelegt ist, dass die Ableitung bei einem normalen, rezirkulationslosen spKt/V-Signalverlauf stets darunter liegt, wird häufig erreicht und überschritten. Liegt eine gewisse Anzahl, z.B. drei oder vier, derartiger Überschreitungen des Schwellwerts S vor, wird dies als Indikator für eine vorliegende Rezirkulation gewertet.

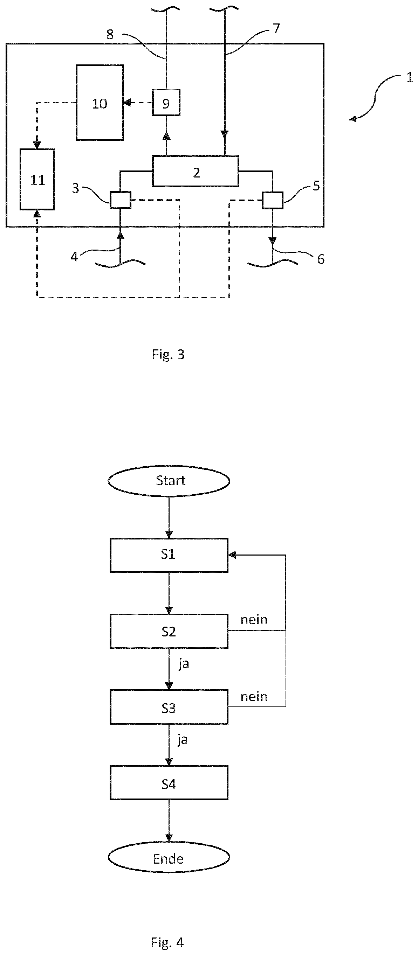

Fig. 3 zeigt schematisch eine extrakorporale Blutbehandlungsmaschine 1. Während einer extrakorporalen Blutbehandlung wird einem Filter 2 oder Dialysator über eine einen arteriellen Drucksensor 3 aufweisende Blutzuleitung 4 Blut eines Patienten zugeführt und wird über eine einen venösen Drucksensor 5 aufweisende Blutableitung 6 das Blut aus dem Filter 2 zurück zu dem Patienten geleitet. Das Blut durchströmt dabei den Filter 2, wobei Schadstoffe aus dem Blut über eine semipermeable Membran in eine im Gegenstromprinzip dazu durch den Filter 2 strömende Dialyseflüssigkeit übergehen. Dazu wird durch eine Dialyseflüssigkeitszuleitung 7 frische Dialyseflüssigkeit in den Filter 2 geleitet und wird die verbrauchte Dialyseflüssigkeit durch eine Dialyseflüssigkeitsableitung 8 aus dem Filter 2 abgeleitet. In der Dialyseflüssigkeitsableitung 8 ist eine Kt/V-Messzelle 9 angeordnet, welche den Kt/V-Signalverlauf während einer extrakorporalen Blutbehandlung vorzugsweise durchgängig misst. Diese Kt/V-Messzelle 9 übermittelt das Kt/V-Signal an eine Überwachungseinrichtung 10 (alternativ kann die Kt/V-Messzelle 9 ein Teil der Überwachungseinrichtung 10 sein), welche anschließend das nachfolgend mit Referenz auf Fig. 4 beschriebene Verfahren durchführt.

Die Überwachungseinrichtung 10 erhält oder ermittelt in Schritt S1 das Kt/V-Signal. In Schritt S2 wird das Signal wie beispielsweise mit Referenz auf Fig. 2a und 2b beschrieben analysiert und ermittelt, ob einer der vorstehend mit Bezug auf Fig. 1a bis 1f beschriebenen Indikatoren für eine vorliegende Rezirkulation vorliegt. Falls kein solcher Indikator ermittelt wird (in Fig. 4 mit "nein" gekennzeichnet), kehrt die Überwachungseinrichtung 10 zu Schritt S1 zurück. Falls jedoch einer der Indikatoren ermittelt wird (in Fig. 4 mit "ja" gekennzeichnet), wird mit Schritt S3 eine Plausibilitätsprüfung durchgeführt, wobei weitere Sensordaten, wie z.B. Messwerte des venösen Drucksensors 5 und ggf. des arteriellen Drucksensors 7, aktuelle Betriebsparameter der extrakorporalen Blutbehandlungsmaschine 1, oder ein Vorliegen weiterer Indikatoren geprüft werden, um festzustellen, ob ein Vorliegen einer Rezirkulation eine plausible Ursache für das Auftreten des in Schritt S2 ermittelten Indikators sein kann. Falls die Plausibilitätsprüfung negativ ist (in Fig. 4 mit "nein" gekennzeichnet), kehrt die Überwachungseinrichtung 10 zu Schritt S1 zurück. Falls die Plausibilitätsprüfung jedoch positiv ist (in Fig. 4 mit "ja" gekennzeichnet), wird mit Schritt S4 ein Trigger oder Auslösesignal ausgegeben, welches entweder direkt an eine Rezirkulationsmesszelle 11 ausgegeben wird, wodurch automatisch eine genaue, zuverlässige Rezirkulationsmessung eingeleitet wird, oder durch welches automatisch, beispielsweise über eine Displayanzeige oder ein hörbares Signal, automatisch eine Aufforderung zum Einleiten einer Rezirkulationsmessung an einen Anwender ausgegeben wird.

### Liste der Bezugszeichen

- 1: Extrakorporale Blutbehandlungsmaschine
- 2: Filter
- 3: Arterieller Drucksensor
- 4: Blutzuleitung
- 5: Venöser Drucksensor
- 6: Blutableitung
- 7: Dialyseflüssigkeitszuleitung
- 8: Dialyseflüssigkeitsableitung
- 9: Kt/V-Messzelle
- 10: Überwachungseinrichtung
- 11: Rezirkulationsmesszelle
- S1: Erfassen des Kt/V-Signals
- S2: Analyse des Kt/V-Signals / Ermittlung eines der Indikatoren
- S3: Plausibilitätsprüfung
- S4: Ausgabe des Auslösesignals

- Kt/V_{Ist}: Gemessenes Kt/V-Signal
- Kt/Vₜₕₑₒ: theoretische Kt/V-Verlauf
- Kt/V_{Soll}: Soll-Kt/V-Wert
- t_{Soll}: Soll-Behandlungszeit

## Patentansprüche

1. Überwachungseinrichtung einer extrakorporalen Blutbehandlungsmaschine, welche dazu ausgelegt ist, ein Signal zu erfassen, das kontinuierlich eine Schadstoffkonzentration in verbrauchter Dialyseflüssigkeit abbildet, und einen Signalverlauf automatisch bezüglich zumindest eines vorbestimmten Signalverlauf-Indikators oder Signalverlauf-Musters für eine vorliegende Rezirkulation auszuwerten,
**dadurch gekennzeichnet, dass** die Überwachungseinrichtung ferner dazu ausgelegt ist,
zur Auswertung das Signal zu differenzieren, um ein Differenzialsignal zu berechnen und mit zumindest einem Schwellwert zu vergleichen, wobei als Signalverlauf-Indikator oder Signalverlauf-Muster für eine vorliegende Rezirkulation eine bestimmte Häufigkeit oder Dauer von Schwellwertüberschreitungen und/oder eine einmalige Schwellwertüberschreitung des gleichen oder eines anderen Schwellwerts erkannt werden bzw. wird und wobei der Schwellwert abhängig ist von der Soll-Behandlungszeit und einem Signalwert einer oder mehrerer früherer extrakorporaler Blutbehandlungen des spezifischen Patienten, und
bei Ermitteln des zumindest einen Signalverlauf-Indikators oder Signalverlauf-Musters automatisch eine Rezirkulationsmessung einzuleiten oder automatisch eine Aufforderung zum Einleiten einer Rezirkulationsmessung an einen Anwender auszugeben.

2. Überwachungseinrichtung nach einem der vorstehenden Ansprüche, wobei das Signal einen Kt/V-Wert abbildet oder ein Rohsignal ist, vorzugsweise Intensitäts- und/oder Absorbtionswerte einer spektroskopischen Messung, auf dessen Basis der Kt/V-Wert berechenbar ist.

3. Überwachungseinrichtung nach Anspruch 1 oder 2, wobei der Schwellwert abhängig ist von einer Soll-Behandlungszeit sowie einem Soll-Signalwert und/oder einem Signalwert einer früheren extrakorporalen Blutbehandlung und/oder einem theoretisch errechneten Signalwert.

4. Überwachungseinrichtung nach einem der vorstehenden Ansprüche, welche dazu eingerichtet ist, aus dem Signal eine erwartete Behandlungszeit zu errechnen, zu welcher ein Soll-Signalwert voraussichtlich erreicht wird, und mit einer Soll-Behandlungszeit zu vergleichen, wobei ein deutliches Unterschreiten oder ein deutliches Überschreiten der Soll-Behandlungszeit als ein Signalverlauf-Indikator oder Signalverlauf-Muster für eine vorliegende Rezirkulation dient.

5. Überwachungseinrichtung nach einem der vorstehenden Ansprüche, welche dazu eingerichtet ist, den Signalverlauf mit bekannten, vorab abgespeicherten Referenz-Mustern oder Patterns eines Signalverlaufs zu vergleichen, welche bei einer vorliegenden Rezirkulation auftreten, und als Signalverlauf-Indikator oder Signalverlauf-Muster für eine vorliegende Rezirkulation eine ausreichender Übereinstimmung des Signals mit den bekannten Referenz-Mustern oder Pattern zu erkennen.

6. Überwachungseinrichtung nach einem der vorstehenden Ansprüche, welche ferner dazu ausgelegt ist, nach dem Ermitteln des zumindest einen Signalverlauf-Indikators oder Signalverlauf-Musters, vorzugsweise unmittelbar danach, eine Plausibilitätsprüfung einzuleiten.

7. Überwachungseinrichtung nach Anspruch 6, welche dazu eingerichtet ist, im Rahmen der Plausibilitätsprüfung das Auftreten des Signalverlauf-Indikators oder Signalverlauf-Musters mit weiteren Sensordaten, vorzugsweise einem venösen Druck, einer Änderung eines Betriebszustands oder Parameters der extrakorporalen Blutbehandlungsmaschine oder einem theoretisch berechneten Signalverlauf zu vergleichen.

8. Überwachungseinrichtung nach einem der Ansprüche 6 und 7, wobei mehrere Signalverlauf-Indikatoren oder Signalverlauf-Muster ermittelt werden und diese im Rahmen der Plausibilitätsprüfung verglichen werden.

9. Extrakorporale Blutbehandlungsmaschine, vorzugsweise Dialysemaschine, mit einer Überwachungseinrichtung nach einem der vorstehenden Ansprüche.

## Claims

1. A monitoring device of an extracorporeal blood treatment machine, which is configured to detect a signal continuously representing a concentration of pollutants in a used dialysis liquid and to automatically evaluate a signal course with respect to at least one predetermined signal course indicator or signal course pattern for an existing recirculation, **characterized in that** the monitoring device is further configured to
differentiate the signal for evaluation in order to calculate a differential signal and compare it with at least one threshold value, wherein a certain frequency or duration of threshold value overruns and/or a one-time threshold value overrun of the same or another threshold value is recognized as a signal course indicator or signal course pattern for an existing recirculation, and
upon determination of the at least one signal course indicator or signal course pattern, to automatically initiate a recirculation measurement or automatically output a request for initiating a recirculation measurement to a user.

2. The monitoring device according to any of the preceding claims, wherein the signal represents a Kt/V value or is a raw signal, preferably intensity and/or absorption values of a spectroscopic measurement on the basis of which the Kt/V value can be calculated.

3. The monitoring device according to claim 1 or 2, wherein the threshold value depends on a desired treatment time as well as a desired signal value and/or a signal value of a previous extracorporeal blood treatment and/or a theoretically calculated signal value.

4. The monitoring device according to any of the preceding claims, which is arranged for calculating, from the signal, an expected treatment time at which a desired signal value is expected to be reached, and for comparing it with a desired treatment time, wherein a clear shortfall or a clear overshoot of the desired treatment time serves as a signal course indicator or signal course pattern for an existing recirculation.

5. The monitoring device according to any of the preceding claims, which is configured for comparing the signal course with known, previously stored reference patterns or patterns of a signal course which occur with an existing recirculation, and to recognize a sufficient correspondence of the signal with the known reference patterns or patterns as a signal course indicator or signal course pattern for an existing recirculation.

6. The monitoring device according to any of the preceding claims, which is further configured to initiate a plausibility check after the determination of the at least one signal course indicator or signal course pattern, preferably immediately thereafter.

7. The monitoring device according to claim 6, which within the framework of the plausibility check is configured for comparing the occurrence of the signal course indicator or signal course pattern with further sensor data, preferably a venous pressure, a change in an operational state or parameter of the extracorporeal blood treatment machine or a theoretically calculated signal course.

8. The monitoring device according to any of claims 6 and 7, wherein several signal course indicators or signal course patterns are determined and compared within the framework of the plausibility check.

9. An extracorporeal blood treatment machine, preferably a dialysis machine, comprising a monitoring device according to any of the preceding claims.

## Revendications

1. Système de surveillance d'une machine de traitement extracorporel du sang qui est conçu afin de détecter un signal qui représente en continu une concentration de substances nocives dans un liquide de dialyse usagé, et d'évaluer un profil de signal automatiquement par rapport à au moins un indicateur de profil de signal prédéterminé ou modèle de profil de signal pour une recirculation existante,
**caractérisé en ce que** le système de surveillance est de plus conçu
pour différencier le signal pour l'évaluation afin de calculer un signal différentiel et de le comparer avec au moins une valeur seuil, dans lequel une fréquence ou durée déterminée de dépassements de valeur seuil et/ou un dépassement de valeur seuil unique de la même valeur seuil ou d'une autre valeur seuil est reconnue comme indicateur de profil de signal ou modèle de profil de signal pour une recirculation existante, et dans lequel la valeur seuil dépend du temps de traitement de consigne et d'une valeur de signal d'un ou de plusieurs traitements extracorporels du sang antérieurs du patient spécifique et
pour introduire automatiquement, lors du calcul de l'au moins un indicateur de profil de signal ou modèle de profil de signal, une mesure de recirculation ou pour délivrer automatiquement en sortie une demande d'introduction d'une mesure de recirculation à un utilisateur.

2. Système de surveillance selon l'une quelconque des revendications précédentes, dans lequel le signal représente une valeur Kt/V ou est un signal brut, de préférence des valeurs d'intensité et/ou d'absorption d'une mesure spectroscopique, sur la base de laquelle la valeur Kt/V peut être calculée.

3. Système de surveillance selon la revendication 1 ou 2, dans lequel la valeur seuil dépend d'un temps de traitement de consigne ainsi que d'une valeur de signal de consigne et/ou d'une valeur de signal d'un traitement extracorporel du sang antérieur et/ou d'une valeur de signal calculée de manière théorique.

4. Système de surveillance selon l'une quelconque des revendications précédentes qui est conçu afin de calculer, à partir du signal, un temps de traitement attendu auquel une valeur de signal de consigne est probablement atteinte, et de le comparer avec un temps de traitement de consigne, dans lequel une non-atteinte nette ou un dépassement net du temps de traitement de consigne sert d'indicateur de profil de signal ou de modèle de profil de signal pour une recirculation existante.

5. Système de surveillance selon l'une quelconque des revendications précédentes qui est conçu afin de comparer le profil de signal avec des modèles de référence ou motifs connus, enregistrés au préalable d'un profil de signal qui surviennent en cas de recirculation existante, et de reconnaître une concordance suffisante du signal avec les modèles de référence ou motifs connus comme indicateur de profil de signal ou modèle de profil de signal pour une recirculation existante.

6. Système de surveillance selon l'une quelconque des revendications précédentes qui est de plus conçu afin introduire une vérification de plausibilité après le calcul d'au moins un indicateur de profil de signal ou de modèle de profil de signal, de préférence directement après.

7. Système de surveillance selon la revendication 6 qui est conçu afin de comparer, dans le cadre de la vérification de plausibilité, la survenue de l'indicateur de profil de signal ou de modèle de profil de signal avec d'autres données de capteur, de préférence une pression veineuse, une modification d'un état de fonctionnement ou d'un paramètre de la machine de traitement extracorporel du sang ou un profil de signal calculé de manière théorique.

8. Système de surveillance selon l'une quelconque des revendications 6 et 7, dans lequel plusieurs indicateurs de profil de signal ou modèles de profil de signal sont calculés et ceux-ci sont comparés dans le cadre de la vérification de plausibilité.

9. Machine de traitement extracorporel du sang, de préférence machine de dialyse, avec un système de surveillance selon l'une quelconque des revendications précédentes.
